# EUROPÄISCHE PATENTSCHRIFT

(11) **EP 2 773 296 B1**
(45) Veröffentlichungstag und Bekanntmachung des Hinweises auf die Patenterteilung: **06.07.2016**
(21) Anmeldenummer: 12790400.1
(22) Anmeldetag: 30.10.2012
(51) Int. Cl.: A61F 2/80, A61F 2/76, A61F 2/50

(54) **SCHAFTSYSTEM FÜR EINE PROTHESE, PROTHESE UND HERSTELLUNGSVERFAHREN**
SHAFT SYSTEM FOR A PROSTHESIS, PROSTHESIS AND PRODUCTION METHOD
SYSTÈME D'EMBOÎTURE POUR UNE PROTHÈSE, PROTHÈSE ET PROCÉDÉ DE FABRICATION

(30) Priorität: 03.11.2011 DE 102011117802
(43) Veröffentlichungstag der Anmeldung: 10.09.2014
(73) Patentinhaber: Otto Bock HealthCare GmbH, 37115 Duderstadt (DE)
(72) Erfinder: GOTTLIEB, Harald, 37345 Jützenbach (DE); VOLKMAR, Marco, 37115 Duderstadt (DE)
(74) Vertreter: Friedrich, Andreas
(86) Internationale Anmeldenummer: PCT/EP2012/004537
(87) Internationale Veröffentlichungsnummer: WO 2013/064241

(56) Entgegenhaltungen:
- US-A- 5 163 965
- US-A- 5 263 990
- US-A- 5 957 980
- US-A- 6 063 125
- US-B1- 6 231 617

## Beschreibung

Die Erfindung betrifft ein Schaftsystem für eine Prothese, wobei das Schaftsystem einen Prothesenschaft, der eine proximale Öffnung zum Aufnehmen eines Amputationsstumpfes und ein distales Ende aufweist, und der zumindest teilweise aus einem ersten Kunststoff besteht, und ein Adapterelement umfasst, das an dem distalen Ende des Prothesenschaftes angeordnet ist und derart ausgebildet ist, dass ein distales Prothesenelement an dem Adapterelement befestigbar ist, wobei das Adapterelement einen Grundkörper aufweist, der zumindest teilweise aus einem zweiten Kunststoff besteht. Die Erfindung betrifft zudem ein Herstellungsverfahren für ein derartiges Schaftsystem sowie eine mit einem derartigen Schaftsystem ausgerüstete Prothese.

Derartige Schaftsysteme sind aus dem Stand der Technik bekannt. Sie weisen einen Prothesenschaft auf, in dessen proximales Ende ein Amputationsstumpf des Patienten eingeführt wird, wenn eine mit einem der-artigen Schaftsystem ausgerüstete Prothese vom Patienten angelegt wird. Um einen optimalen Halt und Sitz zu gewährleisten sowie zur Polsterung des teilweisen empfindlichen Amputationsstumpfes kann über den Amputationsstumpf zunächst ein Liner aus einem elastischen Material, beispielsweise Silikon, gezogen werden. Anschließend wird der somit am Liner versehene Amputationsstumpf in den Prothesenschaft eingeführt. Alternativ dazu ist es auch denkbar, eine Polsterung direkt an dem Prothesenschaft vorzusehen und so auf den Liner oder eine ähnliche Vorrichtung zu verzichten.

Der Prothesenschaft verfügt zudem über ein distales Ende, an dem über ein Adapterelement distale Prothesenelemente angeordnet werden können. Diese sind in der Regel Nachbildungen amputierter Extremitäten, beispielsweise eines Fußes, eines Unterschenkels oder einer Hand. Insbesondere wenn es sich um Beinprothesen handelt, müssen sowohl durch die distalen Prothesenelemente als auch durch den Prothesenschaft teilweise enorme Kräfte aufgenommen werden, wie sie beispielsweise beim Gehen mit der Prothese entstehen. Aus diesem Grund ist es notwendig, dass einerseits der Prothesenschaft selbst stabil und haltbar ausgebildet ist und andererseits eine feste und sichere Verbindung des Prothesenschaftes mit dem distalen Prothesenelement gewährleistet ist.

Um die haltbare und stabile Ausführungsform des Prothesenschaftes zu gewährleisten, besteht dieser zumindest teilweise aus einem ersten faserverstärkten Kunststoff. Nach dem Aushärten ist dieser faserverstärkte Kunststoff haltbar und stabil und gleichzeitig leicht, so dass ein möglichst angenehmes Tragegefühl des Prothesenschaftes erreicht wird.

Um eine besonders sichere Verbindung der distalen Prothesenelemente mit dem Prothesenschaft zu gewährleisten, ist am distalen Ende des Prothesenschaftes ein Adapterelement angeordnet, mit dem die distalen Prothesenelemente verbunden werden können.

Nachteilig ist, dass es trotz dieses aufwändig und sicher wirkenden Aufbaus immer wieder zu Klappergeräuschen innerhalb einer Prothese, insbesondere innerhalb einer Beinprothese, kommt. Dies ist für den Träger der Prothese störend, da beispielsweise ein Gehen mit der Prothese immer mit einer Geräuschentwicklung verbunden ist, durch die zudem permanent offensichtlich wird, dass der Patient eine Prothese trägt. Zudem sorgt ein Klappern in einer Beinprothese für ein ständiges Unsicherheitsgefühl, weil beim Patienten der Eindruck entsteht, die Prothese sei nicht ordnungsgemäß hergestellt oder in fehlerhafter Weise angelegt worden. Gleiches gilt natürlich auch für Armprothesen. Das Vertrauen in die richtige Funktionsweise der Prothese und damit das Selbstbewusstsein des Trägers wird daher teilweise stark beeinträchtigt.

Aus der US 5 957 980 ist ein Schaftsystem gemäß des Oberbegriffs des unabhängigen Anspruchs 1 bekannt. Insbesondere offenbart die US 5 957 980 einen Prothesenschaft, an dessen Ende ein Verstärkungselement angeordnet ist, das beispielsweise aus Carbonelementen bestehen und unterschiedliche Platten und Elemente aufweisen kann. An diesem ist ein herkömmliches Adapterelement angeordnet, das für die Befestigung eines distalen Prothesenelementes ausgebildet ist. Die US 5 163 965 hingegen offenbart einen Prothesenschaft, an dessen distalem Ende ein Adapter- oder Befestigungselement angeordnet wird. Dieses besteht zumindest teilweise aus Metall oder einem starren Schaum.

Die US 6 231 617 B1 befasst sich mit einem Prothesenliner, an dessen Ende ein Basiselement angeordnet wird, das aus einem Metall oder einem Kunststoff bestehen kann. Die US 6 063 125 befasst sich mit dem Problem, ein Ventil in dem Adapterelement des Prothesenschaftes unterzubringen, durch das das Volumen zwischen dem Prothesenschaft und einem sich in dem Schaft befindlichen Amputationsstumpf evakuiert beziehungsweise belüftet werden kann. Dabei werden herkömmliche konventionell ausgebildete aus dem Stand der Technik bekannte distale Adapter verwendet.

Der Erfindung liegt somit die Aufgabe zugrunde, ein Schaftsystem für eine Prothese vorzuschlagen, mit dem das Vertrauen des Trägers der Prothese in die Prothese erhöht und verstärkt wird, das Klappern unterbunden wird und gleichzeitig eine sichere und stabile Befestigung von distalen Prothesenelementen an dem Schaftsystem ermöglicht wird.

Die Erfindung löst die gestellte Aufgabe durch ein Schaftsystem nach dem unabhängigen Anspruch 1, das sich dadurch auszeichnet, dass der zweite Kunststoff ein faserverstärkter Kunststoff ist und das Adapterelement mit dem Prothesenschaft zumindest auch formschlüssig verbunden ist.

Der Erfindung liegt die Erkenntnis zugrunde, dass die störenden und das Unsicherheitsgefühl hervorrufenden Klappergeräusche entstehen, weil das Adapterelement, das bei herkömmlichen Schaftsystemen und Prothesen beispielsweise aus Aluminium hergestellt wird, sich relativ zum Prothesenschaft bewegen kann, da die zumeist formschlüssige Verbindung zwischen den beiden Bauteilen ein relativ großes Spiel beinhaltet.

Dieses entsteht beim Herstellungsprozess des Schaftsystems. Herkömmlicherweise wird eine dafür vorgesehene Form, die beispielsweise individuell an den Amputationsstumpf des Patienten angepasst werden kann, mit dem ersten faserverstärkten Kunststoff in einem nicht ausgehärteten Zustand ausgekleidet. An die vorher am Amputationsstumpf des Patienten ausgemessene Position wird dann das Adapterelement angebracht. Dieses besteht beispielsweise auf Aluminium, was den Vorteil hat, dass es leicht zu bearbeiten und relativ kostengünstig ist und zudem ein geringes Eigengewicht aufweist. Anschließend muss der erste faserverstärkte Kunststoff, der später dem Prothesenschaft bilden soll, ausgehärtet werden. Dazu muss die Temperatur deutlich erhöht werden. Da das Adapterelement und der erste faserverstärkte Kunststoff jedoch unterschiedliche Wärmeausdehnungskoeffizienten haben, kommt es zu einer unterschiedlichen Wärmeausdehnung zwischen dem beispielsweise aus Aluminium bestehenden Adapterelement und dem ersten faserverstärkten Kunststoff. Das Adapterelement dehnt sich dabei stärker aus. Nach dem Abkühlen des Schaftsystems ziehen sich sowohl der erste faserverstärkte Kunststoff als auch das Adapterelement wieder zusammen. Aufgrund des größeren Wärmeausdehnungskoeffizienten des Aluminiums zieht sich das Adapterelement stärker zusammen als der erste faserverstärkte Kunststoff, sodass ein Spiel zwischen dem Adapterelement und dem Prothesenschaft entsteht. Dies führt zu dem unangenehmen und störenden Klappergeräusch. Diese überraschend einfache Erklärung führt zu der erfindungsgemäßen Lösung der gestellten Aufgabe, auch das Adapterelement mit einem Grundkörper auszubilden, der aus einem zweiten faserverstärkten Kunststoff besteht. Auf diese Weise können die Wärmeausdehnungskoeffizienten zwischen dem ersten faserverstärkten Kunststoff, der den Prothesenschaft bilden soll, und dem Adapterelement angeglichen werden, so dass es beim Abkühlen nicht mehr zu einem Spiel zwischen dem Adapterelement und dem dann ausgehärteten Prothesenschaft kommt.

Bei einem herkömmlichen Gießharzverfahren wird das Fasermaterial, dass es mit dem Kunststoff zu ummanteln gilt, auf einen einem Positivmodell des Amputationsstumpfes drapiert und von einem Vakuumschlauch umschlossen. In diesen wird die Kunststoffmatrix unter Unterdruck eingefüllt. Diese Kunststoffmatrix reagiert beim Aushärten exotherm in einem Temperaturbereich von ca. 50°C - 80°C. Aufgrund dieser relativ geringen Reaktionstemperatur ist die Wärmeausdehnung bei diesem Verfahren nahezu vernachlässigbar, so dass auch nur ein kaum nennenswertes Spiel entsteht. Im Gegensatz dazu härten PrePregs, also bereits mit einem Kunstharz oder einem Kunststoff vorimprägnierte Fasern in einem Temperaturbereich beispielsweise zwischen 200°C und 300°C aus. Aufgrund dieser deutlich größeren Temperatur ist der Temperaturunterschied zu dem Temperaturbereich, in dem der Prothesenschaft später verwendet werden soll, so groß, dass es hierzu deutlichen Wärmeausdehnungen und damit zu einem deutlich merkbaren Spiel zwischen den einzelnen Bauteilen kommt.

Vorteilhafterweise enthalten der erste faserverstärkte Kunststoff und der zweite faserverstärkte Kunststoff Fasern aus dem gleichen Material. Da das Dehnungsverhalten insbesondere bei Temperaturänderungen im Wesentlichen durch das Material der Fasern bestimmt wird, kann auf diese Weise ein in den meisten Fällen ausreichende Anpassung der Wärmeausdehnungskoeffizienten erreicht werden. Natürlich können beide Materialien auch den gleichen Kunststoff umfassen. Auf diese Weise kann am einfachsten und sichersten gewährleistet werden, dass sowohl das Adapterelement als auch der Prothesenschaft den gleichen Wärmeausdehnungskoeffizienten haben, so dass ein Spiel zwischen den beiden Bauteilen nach dem Aushärten und Abkühlen sicher vermieden werden kann.

Vorzugsweise ist das Adapterelement mit dem Prothesenschaft zumindest auch stoffschlüssig verbunden. Eine sichere formschlüssige Verbindung ist durch die erfindungsgemäße Ausgestaltung des Grundkörpers des Adapterelementes aus einem zweiten faserverstärkten Kunststoff möglich geworden. Insbesondere müssen keine großen Hinterschneidungen und Vorsprünge vorgesehen werden, um das Adapterelement formschlüssig sicher in der dafür vorgesehenen Position zu halten. Zudem ist es durch die erfindungsgemäße Ausgestaltung möglich, beim Aushärten des ersten faserverstärkten Kunststoffes, der den Prothesenschaft bildet, eine stoffschlüssige Verbindung zum Adapterelement herzustellen. Auf diese Weise wird eine besonders sichere Befestigung des Adapterelementes am Prothesenschaft erreicht, die mit den Werkstoffkombinationen aus dem Stand der Technik nicht möglich ist.

In einer besonders bevorzugten Ausführungsform handelt es sich bei dem ersten und/oder zweiten faserverstärkten Kunststoff um einen glasfaser- oder kohlefaserverstärkten Kunststoff. Diese Materialien sind einfach zu verarbeiten, führen zu einer besonders hohen Stabilität des aus ihnen hergestellten Prothesenschaftes und weisen dennoch ein sehr geringes Eigengewicht auf, so dass ein zumindest teilweise aus ihnen hergestellter Prothesenschaft bzw. der aus ihnen hergestellte Grundkörper des Adapterelementes ein angenehmes Traggefühl und eine relativ geringe Belastung des empfindlichen Amputationsstumpfes gewährleisten. Vorzugsweise ist an dem Adapterelement wenigstens ein Gewindeeinsatz angeordnet, der vorteilhafterweise aus einem wasserunempfindlichen Material, insbesondere aus Edelstahl, besteht. Damit ist es auf eine besonders einfache Weise möglich, distale Prothesenelemente an dem Adapterelement anzuordnen. Sie können einfach in die Gewindeeinsätze eingeschraubt werden. Werden die Gewindeeinsätze aus einem wasserunempfindlichen Material hergestellt, ist es möglich, ein Schaftsystem und damit auch eine Prothese herzustellen, der ein Kontakt mit Wasser nichts ausmacht. Insbesondere ist es möglich, das Schaftsystem und die Prothese ausschließlich aus Bauteilen zu fertigen, die insbesondere nicht rosten oder bei Kontakt mit Wasser sonstige Schäden davon tragen. Damit kann eine Prothese, die mit einem derartigen Schaftsystem ausgestattet ist, beispielsweise auch als Badeprothese verwendet werden und muss nicht beispielsweise zum Duschen, Baden oder Schwimmen abgenommen werden. Dies erhöht den Tragekomfort. Zudem ist es möglich, eine Prothese beispielsweise auch kosmetisch so auszubilden, dass der Prothesenträger selbst im Schwimmbad nicht automatisch und auf den ersten Blick als solcher erkannt werden kann.

In einer bevorzugten Ausgestaltung weist der Grundkörper des Adapterelementes einen Kern aus einem faserfreien Kunststoff auf, der von dem zweiten faserverstärkten Kunststoff umgeben ist. Vorzugsweise handelt es sich dabei um den gleichen Kunststoff. Auf diese Weise ist es möglich, insbesondere die teuren Fasermatten, die zur Herstellung des faserverstärkten Kunststoffes verwendet werden, einzusparen und so die Herstellungskosten des Adapterelementes und damit auch des Schaftsystems zu verringern. Zudem ist es möglich, bei dem Adapterelement auch kleine Teile von Fasermatten, beispielsweise Verschnitt aus früheren Produktionsschritten, zu verwenden, um beispielsweise den faserfreien Kern des Adapterelementes mit ihnen zu umgeben. Natürlich kann das Adapterelement auch vollständig aus dem faserverstärkten Kunststoff bestehen, für den dann ebenfalls kleine Fasermattenreste, wie beispielsweise Verschnitt aus anderen Herstellungsschritten, verwendet werden können. Auch dies reduziert die Herstellungskosten, da der Anteil des Verschnitts an den teuren Fasermatten reduziert wird. Besonders bevorzugt ist der Grundkörper des Adapterelementes als faserverstärktes Spritzgussteil ausgebildet. Unter besonderen Umständen kann auch das vollständige Adapterelement als faserverstärktes Spritzgussteil ausgebildet sein.

Eine erfindungsgemäße Prothese verfügt über ein oben beschriebenes Schaftsystem und weist daher die dort beschriebenen Vorteile auf.

Bei einem erfindungsgemäßen Verfahren zum Herstellen eines Schaftsystems werden insbesondere die folgenden Schritte durchgeführt:
- Ummanteln oder Drapieren einer Form mit dem ersten faserverstärkten Kunststoff in einem nicht ausgehärteten Zustand,
- Anordnen des Adapterelementes mit einem Grundkörper aus einem zweiten faserverstärkten Kunststoff, insbesondere in einem ausgehärteten Zustand, so, dass das Adapterelement in Kontakt mit dem nicht ausgehärteten ersten faserverstärkten Kunststoff ist und
- Aushärten des ersten faserverstärkten Kunststoffes, so dass es zu einer zumindest formschlüssigen Verbindung zwischen dem ersten faserverstärkten Kunststoff und dem Adapterelement kommt.

Besonders bevorzugt liegt der zweite faserverstärkte Kunststoff beim Anordnen des Adapterelementes in einem zumindest teilweise nicht ausgehärteten Zustand vor, so dass es beim Aushärten des ersten faserverstärkten Kunststoffes zu einer zumindest auch stoffschlüssigen Verbindung zwischen dem Adapterelement und dem ersten faserverstärkten Kunststoff kommt. In diesem Fall wird sowohl der erste faserverstärkte Kunststoff als auch der zweite faserverstärkte Kunststoff gleichzeitig in einem Verfahrensschritt vollständig ausgehärtet. Im Verfahrensschritt a) wird herkömmlicherweise auf einem Positiv-Modell des Amputationsstumpfes des Patienten oder einem Standardmodell das Prepreg-Material, also der erste faserverstärkte Kunststoff in dem nicht ausgehärteten Zustand angeordnet beziehungsweise drapiert. Das Modell wird dabei üblicherweise mit dem Material ummantelt.

Mit Hilfe einer Zeichnung wird nachfolgend ein Ausführungsbeispiel der vorliegenden Erfindung näher erläutert. Es zeigt
- Figur 1: - ein Schaftsystem gemäß einem ersten Ausführungsbeispiel der vorliegenden Erfindung in einer schematischen 3D-Ansicht,
- Figur 2: - ein Schaftsystem gemäß einem weiteren Ausführungsbeispiel der vorliegenden Erfindung in einer Schnittdarstellung,
- Figur 3: - ein Schaftsystem gemäß einem weiteren Ausführungsbeispiel der vorliegenden Erfindung in einer Explosionsdarstellung,
- Figur 4: - ein Adapterelement für ein Schaftsystem gemäß ei nem Ausführungsbeispiel der vorliegenden Erfindung in einer schematischen 3D-Ansicht,
- Figur 5: - das Adapterelement aus Figur 4 in einer Explosions darstellung, und
- Figur 6: - eine Prothese gemäß einem Ausführungsbeispiel der vorliegenden Erfindung.

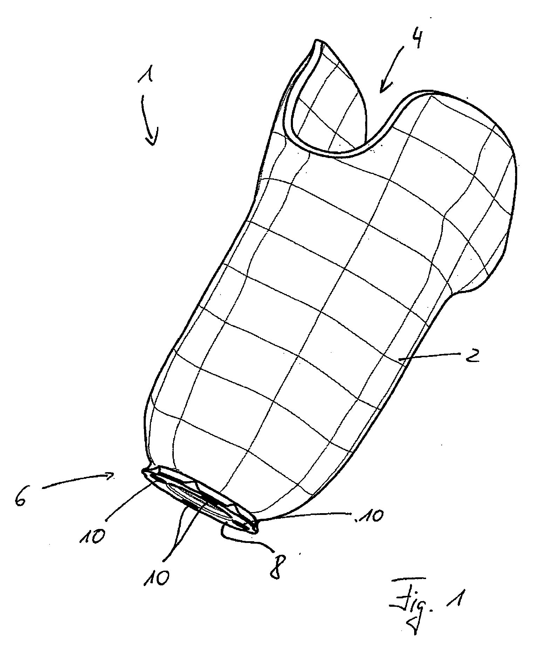

Figur 1 zeigt ein Schaftsystem 1 gemäß einem ersten Ausführungsbeispiel der vorliegenden Erfindung. Man erkennt einen Prothesenschaft 2, der aus einem ersten faserverstärkten Kunststoff hergestellt ist. Am in Figur 1 oberen Ende des Prothesenschaftes 2 ist eine proximale Öffnung 4 dargestellt, in die ein Amputationsstumpf, in diesem Fall ein Unterschenkelstumpf, eingebracht werden kann.

Der Prothesenschaft 2 verfügt zudem über ein distales Ende 6, an dem ein Adapterelement 8 angeordnet ist. Im gezeigten Ausführungsbeispiel besteht der Prothesenschaft 2 vollständig aus dem ersten faserverstärkten Kunststoff. Natürlich ist es auch denkbar, beispielsweise nur einen distalen Anteil aus dem ersten faserverstärkten Kunststoff herzustellen und den verbleibenden Rest des Prothesenschaftes 2 beispielsweise aus einem weicheren oder flexibleren Material herzustellen. Auch ist es denkbar, den Prothesenschaft 2 in der gezeigten Ausführungsform mit Fenstern zu versehen, in denen beispielsweise ein flexibles, elastisches oder weicheres Material vorgesehen sein kann.

In Figur 1 ist zu erkennen, dass am Adapterelement 8 vier Gewindeeinsätze 10 vorgesehen sind, über die ein nicht gezeigtes distales Prothesenelement mit dem Adapterelement 8 und damit mit dem Prothesenschaft 2 verbunden werden kann.

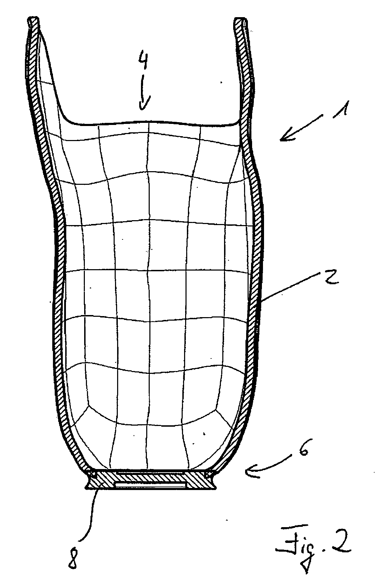

Figur 2 zeigt den Prothesenschaft 2 in einer Schnittdarstellung. Am distalen Ende 6 des Prothesenschaftes 2 befindet sich das Adapterelement 8. Die Gewindeeinsätze 10 sind in Figur 2 nicht gezeigt.

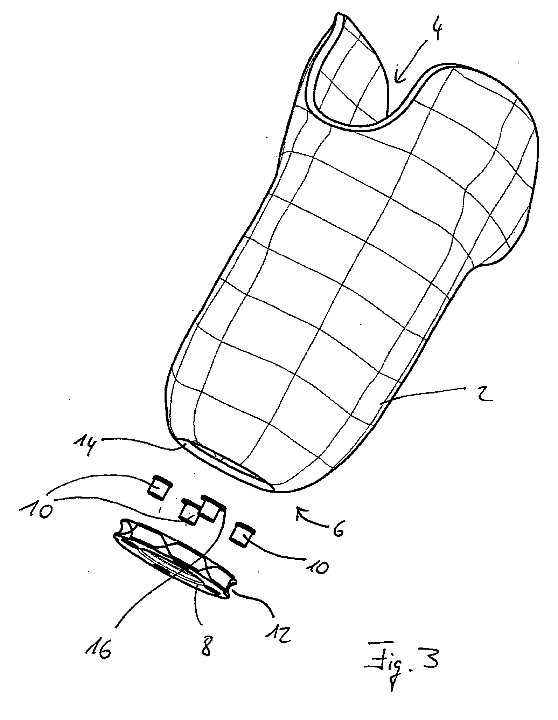

Figur 3 zeigt die Darstellung aus Figur 1 in einer Explosionsdarstellung. Man erkennt am Adapterelement 8 umlaufend eine in Figur 3 relativ flache Nut 12, in die ein Rand 14, der am distalen Ende 6 des Prothesenschaftes 2 vorgesehen ist, eingreift. Damit kommt es zu einer formschlüssigen Verbindung zwischen dem Adapterelement 8 und dem Prothesenschaft 2. Man erkennt zudem, dass die vier Gewindeeinsätze 10 an ihrem der proximalen Öffnung 4 des Prothesenschaftes 2 zugewandten Ende jeweils einen kleinen Flansch 16 aufweisen, der ein Herausziehen der Gewindeeinsätze 10 aus dem Adapterelement 8 in distaler Richtung, in Figur 3 also nach unten, sicher verhindert.

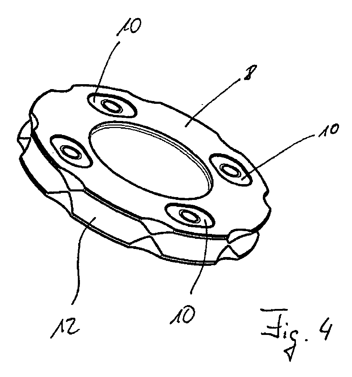

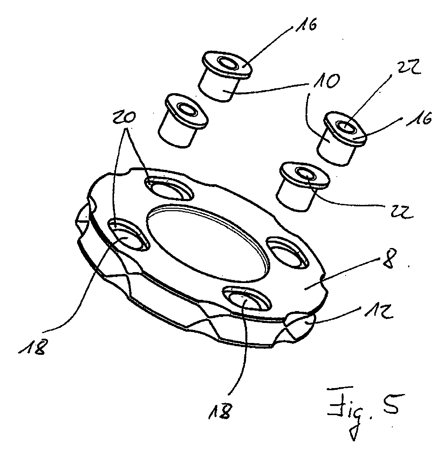

Figur 4 zeigt eine vergrößerte Darstellung des Adapterelementes 8. Figur 5 zeigt die Darstellung aus Figur 4 in einer Explosionsdarstellung. Man erkennt das Adapterelement 8 mit der umlaufenden Nut 12, in die der Rand 14 des Prothesenschaftes 2 eingreift, um so eine formschlüssige Verbindung herzustellen. In Figur 5 sind vier Ausnehmungen 18 dargestellt, in die die vier Gewindeeinsätze 10 eingesetzt werden. An den Gewindeeinsätzen 10 ist am in Figur 5 oberen Ende jeweils deutlich der Flansch 16 zu erkennen, mit dem die Gewindeeinsätze 10 jeweils auf einem in jeder Ausnehmung 18 vorgesehenen Vorsprung 20 aufliegen. Auf diese Weise ist ein Hindurchfallen oder ein Herausziehen der Gewindeeinsätze 10 in Figur 5 nach links unten, also in distaler Richtung, unmöglich. Die Gewindeeinsätze 10 verfügen jeweils über eine zentrale Bohrung 22, die im gezeigten Ausführungsbeispiel eine durchgehende Öffnung ist und mit einem Innengewinde versehen ist. In dieses können Schraubverbindungen eingebracht werden, durch die distale Prothesenelemente mit dem Adapterelement 8 verbunden werden können. Natürlich ist es genauso denkbar, dass die zentralen Bohrungen 22 nicht durchgehend sind, sondern lediglich von der in Figur 5 nicht gezeigten Unterseite der Gewindeeinsätze 10 her zugänglich sind.

In dieser Ausgestaltung ist es möglich, die Gewindeeinsätze 10 bereits in das Adapterelement 8 eingebracht zu haben, bevor das Adapterelement 8 mit dem Prothesenschaft 2 verbunden wird. Alternativ dazu können natürlich auch die Gewindeeinsätze 10 nach dem Einsetzen des Adapterelementes 8 in den Prothesenschaft 2 und insbesondere nach dem Aushärten des ersten faserverstärkten Kunststoffes, der den Prothesenschaft 2 bildet, eingebracht werden.

Die Gewindeeinsätze 10 sind vorteilhafterweise aus einem wasserunempfindlichen Material, beispielsweise Edelstahl, gefertigt. Dieses weist zwar einen anderen Wärmeausdehnungskoeffizienten auf als der umgebende zweite faserverstärkte Kunststoff des Adapterelementes 8, so dass es bei Erwärmen während des Aushärtens der faserverstärkten Kunststoffe zu einer unterschiedlichen Wärmeausdehnung kommt. Diese ist aufgrund der sehr kleinen Maße der Gewindeeinsätze jedoch so klein, dass es zu keinem nennenswerten Spiel kommt. Möchte man jedoch auch dieses noch so kleine Spiel verhindern, ist es ratsam, die Gewindeeinsätze erst nach dem Aushärten der faserverstärkten Kunststoffe und damit erst nach dem Einsetzen des Adapterelementes 8 in den Prothesenschaft 2 einzubringen. Dies ist beispielsweise möglich, indem die Ausnehmungen 18 mit den Vorsprüngen 20 erst nach dem Aushärten der faserverstärkten Kunststoffe und damit nach dem Verbinden des Adapterelementes 8 mit dem Prothesenschaft 2 eingebracht werden.

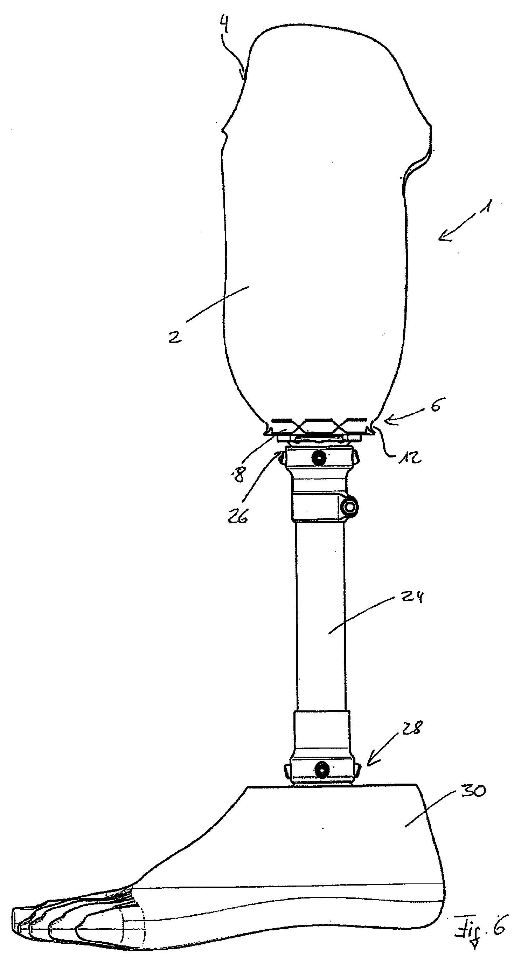

Figur 6 zeigt eine Prothese gemäß einem Ausführungsbeispiel der vorliegenden Erfindung. Man erkennt das Schaftsystem 1 mit dem Prothesenschaft 2 und der proximalen Öffnung 4. Am distalen Ende 6 des Prothesenschaftes 2 ist das Adapterelement 8 angeordnet, an dem die umlaufende Nut 12 zu erkennen ist. An der distalen Seite des Adapterelementes 8 ist ein Prothesenelement angeordnet, das ein Tubuselement 24 umfasst, das an seinem proximalen Ende 26 an dem Adapterelement 8 festgelegt ist. Das Tubuselement 24 kann beispielsweise längenverstellbar ausgebildet sein, um bei Amputationsstümpfen unterschiedlicher Länge einsetzbar zu sein. Am dem proximalen Ende 26 entgegengesetzten distalen Ende 28 befindet sich ein Prothesenfuß 30.

### Bezugszeichenliste

- 1: Schaftsystem
- 2: Prothesenschaft
- 4: Proximale Öffnung
- 6: distales Ende
- 8: Adapterelement
- 10: Gewindeeinsatz
- 12: Nut
- 14: Rand
- 16: Flansch
- 18: Ausnehmung
- 20: Vorsprung
- 22: Bohrung
- 24: Tubuselement
- 26: proximales Ende
- 28: distales Ende
- 30: Prothesenfuß

## Patentansprüche

1. Schaftsystem (1) für eine Prothese, wobei das Schaftsystem (1)
- einen Prothesenschaft (2), der
- eine proximale Öffnung (4) zum Aufnehmen eines Amputationsstumpfes und
- ein distales Ende (6) aufweist, und der
- zumindest teilweise aus einem ersten faserverstärkten Kunststoff besteht, und
- ein Adapterelement (8) umfasst, das
- an dem distalen Ende (6) des Prothesenschaftes (2) angeordnet ist und
- derart ausgebildet ist, dass ein distales Prothesenelement an dem Adapterelement (8) befestigbar ist,
wobei das Adapterelement (8) einen Grundkörper aufweist, der zumindest teilweise aus einem zweiten Kunststoff besteht, **dadurch gekennzeichnet, dass** der zweite Kunststoff ein faserverstärkter Kunststoff ist und das Adapterelement (8) mit dem Prothesenschaft (2) zumindest auch formschlüssig verbunden ist.

2. Schaftsystem (1) nach Anspruch 1, **dadurch gekennzeichnet, dass** der erste faserverstärkte Kunststoff und der zweite faserverstärkte Kunststoff Fasern aus dem gleichen Material enthalten.

3. Schaftsystem (1) nach Anspruch 1 oder 2, **dadurch gekennzeichnet, dass** das Adapterelement (8) mit dem Prothesenschaft (2) zumindest auch stoffschlüssig verbunden ist.

4. Schaftsystem (1) nach einem der vorstehenden Ansprüche, **dadurch gekennzeichnet, dass** der erste faserverstärkte Kunststoff und/oder der zweite faserverstärkte Kunststoff ein glasfaser- oder kohlefaserverstärkter Kunststoff ist.

5. Schaftsystem (1) nach einem der vorstehenden Ansprüche, **dadurch gekennzeichnet, dass** in dem Adapterelement (8) wenigstens ein Gewindeeinsatz (10) angeordnet ist.

6. Schaftsystem (1) nach einem der vorstehenden Ansprüche, **dadurch gekennzeichnet, dass** der wenigstens eine Gewindeeinsatz (10) aus einem wasserunempfindlichen Material, insbesondere aus Edelstahl, besteht.

7. Schaftsystem (1) nach einem der vorstehenden Ansprüche, **dadurch gekennzeichnet, dass** der Grundkörper des Adapterelementes (8) als faserverstärktes Spritzgussteil ausgebildet ist.

8. Prothese mit einem Schaftsystem (1) nach einem der vorstehenden Ansprüche.

9. Verfahren zum Herstellen eines Schaftsystems (1) nach einem der Ansprüche 1 bis 7, mit den Schritten
a) Ummanteln oder Drapieren einer Form mit dem ersten faserverstärkten Kunststoff in einem nicht ausgehärteten Zustand,
b) Anordnen des Adapterelementes (8) aus einem zweiten faserverstärkten Kunststoff, insbesondere in einem ausgehärteten Zustand, so, dass das Adapterelement (8) in Kontakt mit dem nicht ausgehärteten ersten faserverstärkten Kunststoff ist,
c) Aushärten des ersten faserverstärkten Kunststoffes, so dass es zu einer zumindest auch formschlüssigen Verbindung zwischen dem ersten faserverstärkten Kunststoff und dem Adapterelement (8) kommt.

10. Verfahren nach Anspruch 9, wobei beim Anordnen des Adapterelementes (8) der zweite faserverstärkte Kunststoff in einem zumindest teilweise nicht ausgehärteten Zustand vorliegt, wobei der zweite faserverstärkte Kunststoff beim Aushärten des ersten faserverstärkten Kunststoffes mit aushärtet und wobei es zu einer zumindest auch stoffschlüssigen Verbindung zwischen dem Adapterelement (8) und dem ersten faserverstärkten Kunststoff kommt.

## Claims

1. A socket system (1) for a prosthesis, said socket system (1) comprising
- a prosthesis socket (2) which has
- a proximal opening (4) for receiving an amputation stump, and
- a distal end (6), and which
- is made at least partially of a first fiber-reinforced plastic, and
- an adapter element (8) which
- is arranged on the distal end (6) of the prosthesis socket (2) and
- is designed in such a way that a distal prosthetic element can be secured on the adapter element (8),
wherein the adapter element (8) has a main body which is made at least partially of a second plastic **characterized in that** the second plastic is a fiber-reinforced plastic and the adapter element (8) is connected to the prosthesis socket (2) at least also with a form fit.

2. The socket system (1) as claimed in claim 1, **characterized in that** the first fiber-reinforced plastic and the second fiber-reinforced plastic contain fibers of the same material.

3. The socket system (1) as claimed in claim 1 or 2, **characterized in that** the adapter element (8) is connected to the prosthesis socket (2) at least also with cohesive material bonding.

4. The socket system (1) as claimed in one of the preceding claims, **characterized in that** the first fiber-reinforced plastic and/or the second fiber-reinforced plastic is a glass-fiber-reinforced or carbon-fiber-reinforced plastic.

5. The socket system (1) as claimed in one of the preceding claims, **characterized in that** at least one threaded insert (10) is arranged in the adapter element (8).

6. The socket system (1) as claimed in one of the preceding claims, **characterized in that** the at least one threaded insert (10) is made of a material not sensitive to water, in particular of stainless steel.

7. The socket system (1) as claimed in one of the preceding claims, **characterized in that** the main body of the adapter element (8) is designed as a fiber-reinforced injection-molded part.

8. A prosthesis with a socket system (1) as claimed in one of the preceding claims.

9. A method for producing a socket system (1) as claimed in one of claims 1 through 7 with the steps of:
a) sheathing or draping a mold with the first fiber-reinforced plastic in an uncured state,
b) applying the adapter element (8) made of a second fiber-reinforced plastic, in particular in a cured state, such that the adapter element (8) is in contact with the uncured first fiber-reinforced plastic, and
c) curing the first fiber-reinforced plastic, such that an at least also form-fit connection is effected between the first fiber-reinforced plastic and the adapter element (8).

10. The method as claimed in claim 9, wherein, when applying the adapter element (8), the second fiber-reinforced plastic is present in an at least partially uncured state, wherein the second fiber-reinforced plastic cures along with the curing of the first fiber-reinforced plastic, and wherein an at least also materially cohesive bond is effected between the adapter element (8) and the first fiber-reinforced plastic.

## Revendications

1. Système à tige (1) pour une prothèse, dans lequel le système à tige (1) comprend
- une tige de prothèse (2), qui
- comporte une ouverture proximale (4) pour recevoir un moignon d'amputation, et
- une extrémité distale (6), et qui
- est constituée au moins partiellement d'une première matière plastique renforcée par des fibres, et
- inclut un élément adaptateur (8), qui
- est agencé à l'extrémité distale (6) de la tige de prothèse (2) et
- est réalisé de telle façon qu'un élément de prothèse distal est susceptible d'être fixé à l'élément adaptateur (8),
dans lequel l'élément adaptateur (8) comprend un corps de base qui est constitué au moins partiellement d'une seconde matière plastique,
**caractérisé en ce que** la seconde matière plastique est une matière plastique renforcée par des fibres et l'élément adaptateur est relié à la tige de prothèse (2) au moins également à coopération de formes.

2. Système à tige (1) selon la revendication 1, **caractérisé en ce que** la première matière plastique renforcée par des fibres et la seconde matière plastique renforcée par des fibres contiennent des fibres du même matériau.

3. Système à tige (1) selon la revendication 1 ou 2, **caractérisé en ce que** l'élément adaptateur (8) est relié avec la tige de prothèse (2) au moins également à coopération de matières.

4. Système à tige (1) selon l'une des revendications précédentes, **caractérisé en ce que** la première matière plastique renforcée par des fibres et/ou la seconde matière plastique renforcée par des fibres est une matière plastique renforcée par des fibres de verre ou par des fibres de carbone.

5. Système à tige (1) selon l'une des revendications précédentes, **caractérisé en ce qu'**au moins un insert à pas de vis (10) est agencé dans l'élément adaptateur (8).

6. Système à tige (1) selon l'une des revendications précédentes, **caractérisé en ce que** ledit au moins un insert à pas de vis (10) est constitué en un matériau insensible vis-à-vis de l'eau, en particulier en acier inoxydable.

7. Système à tige (1) selon l'une des revendications précédentes, **caractérisé en ce que** le corps de base de l'élément adaptateur (8) est réalisé sous forme de pièce coulée par injection et renforcée par des fibres.

8. Prothèse avec un système à tige (1) selon l'une des revendications précédentes.

9. Procédé pour la réalisation d'un système à tige (1) selon l'une des revendications 1 à 7, avec les étapes consistant à :
a) envelopper ou draper un moule avec une première matière plastique renforcée par des fibres, dans un état non durci,
b) agencer l'élément adaptateur (8) en une matière plastique renforcée par des fibres, en particulier dans un état durci, de telle sorte que l'élément adaptateur (8) est en contact avec la première matière plastique renforcée par des fibres non durcie,
c) faire durcir la première matière plastique renforcée par des fibres, de sorte qu'il se produit une liaison au moins également à coopération de formes entre la première matière plastique renforcée par des fibres et l'élément adaptateur (8).

10. Procédé selon la revendication 9, dans lequel, lors de l'agencement de l'élément adaptateur (8) la seconde matière plastique renforcée par des fibres se présente au moins partiellement dans un état non durci, dans lequel
la seconde matière plastique renforcée par des fibres est durcie conjointement lors du durcissement de la première matière plastique renforcée par des fibres et
il se produit une liaison au moins également à coopération de matières entre l'élément adaptateur (8) et la première matière plastique renforcée par des fibres.
